(19) Europäisches Patentamt
European Patent Office
Office européen des brevets

(11) Veröffentlichungsnummer: **0 111 120**
B1

(12) **EUROPÄISCHE PATENTSCHRIFT**

(45) Veröffentlichungstag der Patentschrift: **17.12.86**

(51) Int. Cl.⁴: **C 07 C 46/02,** C 07 C 50/18, C 07 C 50/24

(21) Anmeldenummer: **83110593.7**

(22) Anmeldetag: **24.10.83**

(54) **Verfahren zur Herstellung von Anthrachinonen.**

(30) Priorität: **10.11.82 DE 3241449**

(43) Veröffentlichungstag der Anmeldung: **20.06.84 Patentblatt 84/25**

(45) Bekanntmachung des Hinweises auf die Patenterteilung: **17.12.86 Patentblatt 86/51**

(84) Benannte Vertragsstaaten: **BE CH DE FR GB IT LI**

(56) Entgegenhaltungen:
**DE-A-2 031 430**
**GB-A-394 990**
**GB-A-2 013 664**

**CHEMICAL ABSTRACTS, Band 50, Nr. 6, 25. März 1956, Spalte 4080 h-i, Columbus, Ohio, US; E. BENGTSSON: "Oxidation of diphenylmethane and benzyltoluene with dilute nitric acid at high temperature and pressure"**

(73) Patentinhaber: **BASF Aktiengesellschaft, Carl-Bosch- Strasse 38, D-6700 Ludwigshafen (DE)**

(72) Erfinder: **Markert, Juergen, Dr., Am Speyerweg 26, D-6704 Mutterstadt (DE)**
Erfinder: **Hagen, Helmut, Dr., Max- Slevogt-Strasse 17 E, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 111 120 B1

LIBER, STOCKHOLM 1986

**Beschreibung**

Die Erfindung betrifft ein Verfahren zur Herstellung von Anthrachinonen durch Oxidation von (o-Dihalogenmethyl)-diphenyl-dihalogenmethanverbindungen mit Salpetersäure in Gegenwart von Schwefelsäure bei erhöhter Temperatur.

Es ist bekannt, an der Phenylgruppen unsubstituierte, in o-Stellung an einer Phenylgruppe eine aliphatische Gruppe tragende Diphenylmethanverbindungen durch Oxidation mit Sauerstoff in der Gasphase in Gegenwart von Vanadium-V-Verbindungen und gegebenenfalls Zusatzmetallverbindungen zu Anthrachinon umzusetzen; Ausbeuten bis zu 55 % werden erhalten. Weder die Methangruppe noch die o-Methylgruppe sind durch Halogenatome substituiert (DE-PS 20 50 798). Eine entsprechende Umsetzung mit Titandioxid und Vanadiumpentoxid als Katalysatoren zeigt Ausbeuten bis zu 69,2 % (DE-OS 24 30 567) oder mit Vanadium, Titan- und Zusatzmetallverbindungen Ausbeuten bis zu 64 % (DE-OS 24 42 911). An den Phenylgruppen halogenierte Diphenylmethanverbindungen werden in Gegenwart von Vanadiumpentoxid und Titandioxid und/oder Zirkondioxid mit Ausbeuten bis zu 73,5 % zu halogenierten Anthrachinonen umgesetzt (DE-OS 25 47 655).

Es ist aus der DE-OS 19 15 385 bekannt, daß man Indane mit Salpetersäure bei 50 bis 250°C und einer Ausbeute bis zu 79,8 % zu o-Benzoylbenzoesäure umsetzt. Es wird angegeben, daß man diese Säure in Schwefelsäure bei 150°C zu Anthrachinon cyclisieren kann. Wie die Beispiele 3 und 9 zeigen, erzielt man so Ausbeuten an Anthrachinon von 62 - 66 % der Theorie.

Es wurde nun gefunden, daß man Anthrachinone der Formel 1,

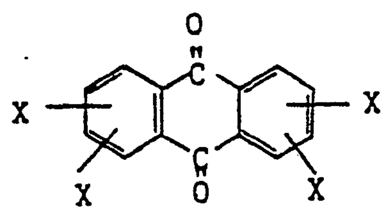

I,

worin die einzelnen Reste R gleich oder verschieden sein können und jeweils ein Halogenatom oder ein Wasserstoffatom bedeuten, durch Oxidation von Diphenylmethanverbindungen bei erhöhter Temperatur vorteilhaft erhält, wenn man (o-Dihalogen-methyl)-diphenyl-dihalogenmethanverbindungen der Formel II,

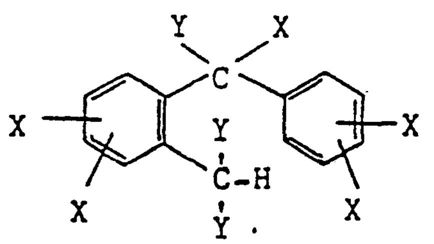

II,

worin X die sogenannte Bedeutung besitzt und die einzelnen Reste Y gleich oder verschieden sein können und jeweils ein Halogenatom bedeuten, mit Salpetersäure in Gegenwart von Schwefelsäure oxidiert.

Die Umsetzung läßt sich für den Fall der Verwendung von (o-Dichlormethyl)-diphenyl-dichlor-methan durch die folgenden Formeln wiedergeben:

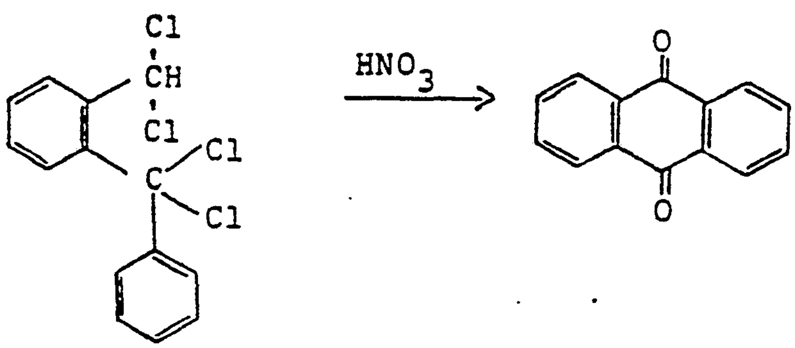

Im Vergleich zum Stand der Technik liefert das Verfahren nach der Erfindung auf einfachere und wirtschaftlichere Weise Anthrachinone in besserer Ausbeute und Reinheit, insbesondere mit Bezug auf in Wasser und Alkalien unlösliche Nebenstoffe. Diese vorteilhaften Ergebnisse sind mit Bezug auf den Stand der Technik überraschend, denn man hätte eine Änderung der Reaktionsrichtung und der Reaktivität der Ausgangsstoffe II, z.B. Bildung von halogensubstituierten o-Phthalsäurederivaten oder entsprechend substituierten Diphenylmethancarbonsäuren oder eine völlige oder teilweise Blockierung der Oxidation und die Bildung von Gemischen schwer trennbarer Nebenprodukte infolge der Beeinflussung der Oxidation durch die Halogensubstituenten erwarten sollen. Daneben war eine teilweise Abspaltung des Halogens und gegebenenfalls erneute Anlagerung des abgespalteten Halogens an anderer Stelle des Moleküls zu vermuten. Man hätte gleichfalls infolge der Verwendung von Salpetersäure einen zusätzlichen, wesentlichen Anteil an nitrierten Verbindungen erwarten sollen. Eine Bildung heterogener Gemische hätte andererseits eine großtechnische, einfache und wirtschaftliche Durchführung des Verfahrens nicht erlaubt. Es ist überraschend, daß man im Vergleich zu den bekannten Verfahren keine Katalysatoren benötigt und im allgemeinen bei wesentlich niedrigeren Temperaturen umsetzt.

Die Ausgangsstoffe II können in bekannter Weise hergestellt werden, z.B. durch Halogenierung von o-Benzyltoluol oder seinen Halogenderivaten nach den in Houben-Weyl, Methoden der org. Chemie, Band 5/3, Seite 735-747 (4. Auflage, Georg-Thieme-Verlag, Stuttgart) beschriebenen Verfahren, zweckmäßig während 2 bis 20 Stunden bei 160 bis 270°C, z.B. bei 180°C. Diese Herstellung ist im allgemeinen praktisch quantitativ und das anfallende Rohprodukt, das im allgemeinen eine Reinheit von ca. 98 % besitzt, kann ohne einen Reinigungsschritt sofort zur Antrachinondarstellung eingesetzt werden. Ein reines Produkt kann durch Destillation oder durch Umkristallisation leicht erhalten werden.

o-Benzyl-toluol und seine Derivate können durch Umsetzung von Benzylchlorid und 3-Chlor-toluol analog den in Ber., Band 6 (1873), Seite 906 beschriebenen Verfahren oder nach den bekannten Verfahren durch Umsetzung von o-Xylylhalogeniden mit Benzolen in Gegenwart von Formelbindungen (DE-OS 23 36 289) oder von Schwefelsäure (DE-OS 24 08 529; 24 29 194) Phosphorsäure (DE-OS 24 28 197; 24 56 747) Aluminium- oder Siliciumverbindungen (DE-OS 25 47 030). Bevorzugte Ausgangsstoffe II und dementsprechend bevorzugte Anthrachinone 1 sind solche, in deren Formeln die einzelnen Reste X und Y gleich oder verschieden sein können und jeweils ein Bromatom oder ein Fluoratom oder insbesondere ein Chloratom bezeichnen, oder darüber hinaus die Reste X auch jeweils ein Wasserstoffatom bedeuten können.

Beispielsweise kommen folgende Verbindungen als Ausgangsstoffe II in Frage: (2-Dichlormethyl-diphenyl)-dichlotmethan; die 3-Chlor-, 4-Chlor-, 5-Chlor-, 3,3'-Dichlor-, 4,4'-Dichlor-, 5,5'-Dichlor-, 6,6'-Dichlor-, 2'-Chlor-, 3'-Chlor-, 4'-Chlor-, 4,6-Dichlor-, 3,4-Dichlor-, 3,5-Di-chlor-, 3,6-Dichlor-, 5,6-Dichlor-, 2,4'-Dichlor-, 2',6'-Dichlor-, 3',5'-Dichlor-, 4,6,4'-Trichlor-, 6,2',4'-Trichlor-, 4,2',4'-Trichlor-, 4,6,2',4'-Tetrachlor-diphenylverbindungen von (2-Dichlornethyl-diphenyl)-dichlormethan; entsprechende an einen oder beiden Phenylgruppen unsubstituierte bzw. Brom oder Fluor substituierte (2-Difluormethyl-diphenyl)-di-fluormethane umd (2-Dibrommethyl-diphenyl)-dibrommethane.

Die Umsetzung wird mit Salpetersäure, zweckmäßig bei einer Temperatur von 50 bis 150°C, vorzugsweise von 90 bis 220°C, durchgeführt. Man arbeitet drucklos oder unter Druck, in allgemeinen bei dem sich unter den Reaktionsbedingungen einstellenden Druck. Die Reaktion kann kontinuierlich oder diskontinuierlich durchgeführt werden.

Man setzt den Ausgangsstoff II mit Salpetersäure in stöchiometrischer Menge oder in Überschuß, vorzugsweise in einem Verhältnis von 30 bis 300, insbesondere 30 bis 60 gew.% Salpetersäure (ber. 100 %), bezogen auf Ausgangsstoff II, um. Im allgemeinen verwendet man für die Oxydation wäßrige Salpetersäurelösungen von 5 bis 99, vorzugsweise von 5 bis 80, insbesondere von 60 bis 70 gew.-%ige Salpetersäure. Die für eine bestimmte Temperatur optimale Salpetersäurekonzentration und Salpetersäuremenge ist durch Vorversuche leicht festzulegen. Bei der Umsetzung werden zweckmäßig von 500 bis 2000, insbesondere 800 bis 1000 Gew.% Schwefelsäure (ber. 100 %), bezogen auf die Gewichtsmenge Ausgangsstoff II, verwendet; bevorzugt kommen 40 bis 95, insbesondere 60 bis 90 gew.-%ige, wäßrige Schwefelsäurelösungen in Betracht.

Die Reaktion kann wie folgt durchgeführt werden: Ein Gemisch von Ausgangsstoff II, Schwefelsäure und Salpetersäure der vorgenannten Konzentration wird 6 bis 11 Stunden bei der Reaktionstemperatur und bei den Reaktionsdruck gehalten. Dann wird das Reaktionsgemisch abgekühlt. Aus ihm kann der Endstoff in üblicher Weise, z.B. durch Filtration oder Dekantieren und Wäsche mit z.B. Methanol, isoliert werden.

Die nach den Verfahren der Erfindung herstellbaren Verbindungen sind wertvolle Ausgangsstoffe für zahlreiche Synthesen (siehe z.B. Ullmanns Encyklopädie der technischen Chemie, Band 3, Seiten 660 - 732 und vorgenannte Veröffentlichungen). Von besonderer Bedeutung sind sie als Ausgangsstoffe für die Herstellung von substituierten Anthrachinonen.

**Beispiel 1**

a) (Herstellung des Ausgangsstoffs) 182 g o-Benzyltoluol wurden in einer Chlorierungsapparatur auf 180°C erhitzt. Bei dieser Temperatur wurde solange Chlor eingeleitet, bis nach gaschromatografischer Kontrolle ein praktisch quantitativer Umsatz zum Ausgangsstoff II stattgefunden hat. Während des Abkühlens wurde das

Gemisch mit Stickstoff gespült und bei 110°C abgetrennt. Es wurden 320 g (o-Dichlormethyl-diphenyl)-dichlormethan vom Schmelzpunkt 93°C und einer Reinheit von 98 % bzw. 305 g reines (o-Dichlormethyl-diphenyl)-dichlormethan (95 % der Theorie) von Schmelzpunkt 97°C (Ligroin) erhalten.

b) (Umsetzung) 160 g (o-Dichlormethyl-diphenyl)-dichlormethan (Beispiel 1a) wurden bei 100 - 110°C in 1,3 kg 70 gew.-%iger Schwefelsäure gelöst. Bei 120°C wurden 48 g 65 gew.-%ige Salpetersäure zur Lösung zugetropft und das Gemisch 8 Stunden bei 125°C gerührt. Die Lösung wurde abgekühlt und der ausgefallene Feststoff abgesaugt. Die Rohausbeute war praktisch quantitativ und die Reinheit 94 %ig. Der Endstoff wurde mit Methanol gewaschen, 94 g (90 % der Theorie) Antrachinon von Schmelzpunkt 286°C wurden erhalten. Die Reinheit war größer als 99 %.

**Beispiel 2**

a) (Herstellung des Ausgangsstoffs) Die Chlorierung wurde analog Beispiel la durchgeführt, wobei das Rohgenisch von 110°C direkt in die auf 100 - 110°C erhitzte Menge an Schwefelsäure (Beispiel 2b) eingetragen wurde.

b) (Umsetzung) Die Umsetzung wurde analog Beispiel 1b mit dem Reaktionsgemisch von Beispiel 2a durchgeführt. Man erhält Anthrachinon vom Fp 286°C, in einer Ausbeute von 90 % der Theorie und einer Reinheit > 99 %.

**Beispiel 3**

32 g (o-Dichlormethyl-diphenyl)-dichlormethan (Beispiel 1a) wurden in 200 ml 85 gew.-%iger Schwefelsäure bei 110°C eingetragen und bei 125°C 7 ml 65 gew.-%ige Salpetersäure zugegeben. Das Gemisch wurde 5 Stunden gerührt. Die Lösung wurde abgekühlt, mit Wasser versetzt und der ausgefallene Feststoff abgesaugt und mit Methanol gewaschen. Es wurden 19 g (91 % der Theorie) Anthrachinon von Fp 286°C erhalten. Die Reinheit war größer als 99 %.

**Patentansprüche**

Verfahren zur Herstellung von Anthrachinonen der Formel I,

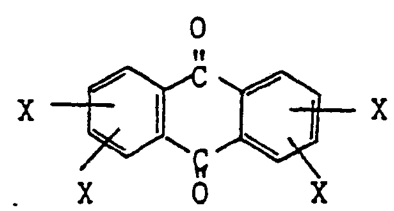

I,

worin die einzelnen Reste X gleich oder verschieden sein können und jeweils ein Halogenatom oder ein Wasserstoffatom bedeuten, durch Oxidation von Diphenylmethanverbindungen bei erhöhter Temperatur, <u>dadurch gekennzeichnet</u>, daß man (o-Dihalogenmethyl)-diphenyl-dihalogemmethanverbindungen der Formel II

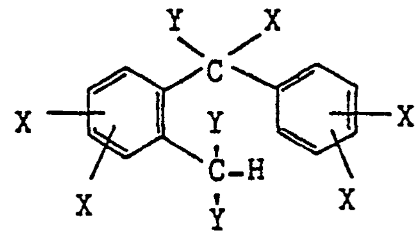

II,

worin X die vorgenannte Bedeutung besitzt und die einzelnen Reste Y gleich oder verschieden sein können und jeweils ein Halogenatom bedeuten, mit Salpetersäure in Gegenwart von Schwefelsäure oxidiert.

**Claim**

A process for the preparation of an anthraquinone of the formula I

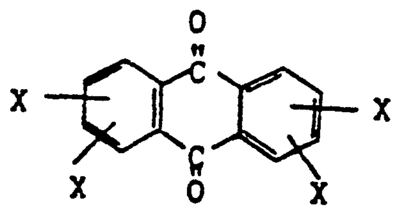

I,

where the individual radicals X may be identical or different and each denotes a halogen or hydrogen atom, by oxidation of a diphenylmethane compound at elevated temperature, wherein an (o-dihalomethyl)-diphenyldihalomethane compound of the formula II

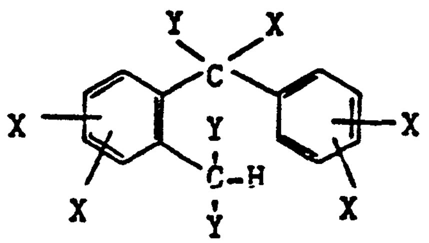

II,

where X has the above meanings, and the individual radicals Y may be identical or different and each denotes a halogen atom, is oxidized with nitric acid in the presence of sulphuric acid.

**Revendication**

Procédé pour la préparation d'anthraquinones de formule I

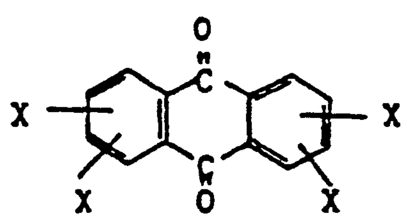

I,

dans laquelle les différents radicaux X peuvent être identiques ou dissemblables et représentent chacun un atome d'halogène ou un atome d'hydrogène, par oxydation de composés de diphénylméthane à température élevée, caractérisé en ce qu'en oxyde avec de l'acide nitrique, en présence d'acide sulfurique, des composés d'(o-dihalogénométhyl)-diphényl-dihalogénométhane de formule II

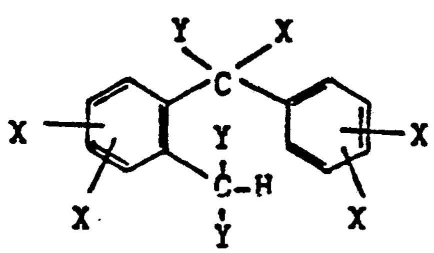

II,

dans laquelle X a la signification donnée ci-dessus et les différents radicaux Y peuvent être identiques ou dissemblables et représentent chacun un atome d'halogène.